# EUROPEAN PATENT SPECIFICATION

(11) **EP 4 281 000 B1**
(45) Date of publication and mention of the grant of the patent: **16.09.2026**
(21) Application number: 22823628.7
(22) Date of filing: 01.12.2022
(51) Int. Cl.: A61B 34/37, A61B 90/00, A61B 34/20, A61F 9/007, A61B 34/10

(54) **VIRTUAL TOOLS FOR MICROSURGICAL PROCEDURES**
VIRTUELLE WERKZEUGE FÜR MIKROCHIRURGISCHE EINGRIFFE
OUTILS VIRTUELS POUR PROCÉDURES MICROCHIRURGICALES

(30) Priority: 02.12.2021 US 202163285185 P
(43) Date of publication of application: 29.11.2023
(73) Proprietor: Forsight Robotics Ltd., Caesarea 3079566 (IL)
(72) Inventor: GOLAN, Yoav, 6219311 Tel Aviv (IL); KORMAN, Tal, 6936059 Tel Aviv (IL); GIL, Ariel, 3258402 Haifa (IL); ARNOLD, Ofer, 2012900 2012900 Ma'ale Zvia (IL); GLOZMAN, Daniel, 4034723 Kfar Yona (IL)
(74) Representative: Evens, Paul Jonathan
(86) International application number: PCT/IB2022/061634
(87) International publication number: WO 2023/100124

(56) References cited:
- WO-A1-2017/044965
- US-A1- 2011 306 986
- US-A1- 2014 114 480
- US-A1- 2021 068 911

## Description

### CROSS-REFERENCES TO RELATED APPLICATIONS

The present application claims priority from U.S. Provisional Patent Application No. 63/285,185 to Golan, filed December 02, 2021, entitled "Virtual tools for microsurgical procedures".

### FIELD OF EMBODIMENTS OF THE INVENTION

Some applications of the present invention generally relate to medical apparatus. Specifically, some applications of the present invention relate to apparatus for performing microsurgical procedures in a robotic manner.

### BACKGROUND

Cataract surgery involves the removal of the natural lens of the eye that has developed an opacification (known as a cataract), and its replacement with an intraocular lens. Such surgery typically involves a number of standard steps, which are performed sequentially.

In an initial step, the patient's face around the eye is disinfected (typically, with iodine solution), and their face is covered by a sterile drape, such that only the eye is exposed. When the disinfection and draping has been completed, the eye is anesthetized, typically using a local anesthetic, which is administered in the form of liquid eye drops. The eyeball is then exposed, using an eyelid speculum that holds the upper and lower eyelids open. One or more incisions (and typically two or three incisions) are made in the cornea of the eye. The incision(s) are typically made using a specialized blade, which is called a keratome blade. At this stage, lidocaine is typically injected into the anterior chamber of the eye, in order to further anesthetize the eye. Following this step, a viscoelastic injection is applied via the corneal incision(s). The viscoelastic injection is performed in order to stabilize the anterior chamber and to help maintain eye pressure during the remainder of the procedure, and also in order to distend the lens capsule.

In a subsequent stage, known as capsulorhexis, a part of the anterior lens capsule is removed. Various enhanced techniques have been developed for performing capsulorhexis, such as laser-assisted capsulorhexis, zepto-rhexis (which utilizes precision nano-pulse technology), and marker-assisted capsulorhexis (in which the cornea is marked using a predefined marker, in order to indicate the desired size for the capsule opening).

Subsequently, it is common for a fluid wave to be injected via the corneal incision, in order to dissect the cataract's outer cortical layer, in a step known as hydrodissection. In a subsequent step, known as hydrodelineation, the outer softer epi-nucleus of the lens is separated from the inner firmer endo-nucleus by the injection of a fluid wave. In the next step, ultrasonic emulsification of the lens is performed, in a process known as phacoemulsification. The nucleus of the lens is broken initially using a chopper, following which the outer fragments of the lens are broken and removed, typically using an ultrasonic phacoemulsification probe. Further typically, a separate tool is used to perform suction during the phacoemulsification. When the phacoemulsification is complete, the remaining lens cortex (i.e., the outer layer of the lens) material is aspirated from the capsule. During the phacoemulsification and the aspiration, aspirated fluids are typically replaced with irrigation of a balanced salt solution, in order to maintain fluid pressure in the anterior chamber. In some cases, if deemed to be necessary, then the capsule is polished. Subsequently, the intraocular lens (IOL) is inserted into the capsule. The IOL is typically foldable and is inserted in a folded configuration, before unfolding inside the capsule. At this stage, the viscoelastic is removed, typically using the suction device that was previously used to aspirate fluids from the capsule. If necessary, the incision(s) is sealed by elevating the pressure inside the bulbus oculi (i.e., the globe of the eye), causing the internal tissue to be pressed against the external tissue of the incision, such as to force closed the incision.

WO 2017/044965 relates to systems and methods for arbitrary viewpoint robotic manipulation and robotic surgical assistance, and discloses apparatus displaying a virtual tool controllable by a user for use to control a robotic tool within the actual environment.

US 2021/306986 relates to a surgical robot sysem using augmented reality, and a method for controlling same.

US 2021/068911 relates to robot-assisted driving systems and methods.

US 2014/114480 relates to methods, devices and systems for non-mechanically restricting and/or programming movement of a tool of a manipulator along a single axis.

### SUMMARY

In accordance with a first aspect of the present invention, there is provided apparatus as defined in appended independent claim 1. Embodiments of the present invention are defined in appended claims dependent on independent claim 1. In accordance with a second aspect of the present invention, there is provided a computer software product as defined in appended independent claim 14.

In accordance with some applications of the present disclosure, a robotic system is configured for use in a microsurgical procedure, such as intraocular surgery. Typically, when used for intraocular surgery, the robotic system includes one or more robotic units (which are configured to hold tools), in addition to an imaging system, one or more displays, and a control-component unit (for example, a control-component unit that includes a pair of control components, such as joysticks), via which one or more operators (e.g., healthcare professionals, such as a physician and/or a nurse) are able to control robotic units. Typically, the robotic system includes one or more computer processors, via which components of the system and the operators operatively interact with each other.

For some applications, a set of tools is provided, each of which includes a universal mount-engagement portion for engaging a tool mount of an end effector of the robotic unit, in accordance with some applications of the present invention. For some applications, the set of tools comprises a universal tool kit for use with the robotic unit that includes all tools that are typically used in a cataract procedure, a different ophthalmic procedure, and/or a different microsurgical procedure. For example, the set of tools typically includes one or more of the following tools: a keratome blade, an eye fixator, a paracentesis knife, a dispersive ophthalmic viscosurgical device (OVD) syringe, a cohesive ophthalmic viscosurgical device (OVD) syringe, a staining syringe (e.g., for staining the anterior lens with a stain such as trypan blue ophthalmic solution), a lidocaine syringe, forceps, a hydrodissection syringe, a phacoemulsification probe, a chopper, an irrigation/aspiration probe, an intraocular lens injector, an antibiotics syringe, and/or a Limbal Relaxing Incision (LRI) knife. For some applications, each of the tools includes one or more markers, which may be used to identify the tools and/or to determine the position and/or orientation of the tool.

Typically, movement of the robotic units (and/or control of other aspects of the robotic system) is at least partially controlled by the one or more operators. For example, the operator may receive images of the patient's eye and the robotic units and/or tools disposed therein, via a display. Based on the received images, the operator typically performs steps of the procedure. For some applications, the operator provides commands to the robotic units via a control component. Typically, such commands include commands that control the position and/or orientation of tools that are disposed within the robotic units, and/or commands that control actions that are performed by the tools. For example, the commands may control a blade, a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the operator may input commands that control the imaging system (e.g., the zoom, focus, and/or x-y positioning of the imaging system). For some applications, the commands include controlling an intraocular-lens-manipulator tool, for example, such that the tool manipulates the intraocular lens inside the eye for precise positioning of the intraocular lens within the eye.

Typically, the control-component unit includes one or more control components, e.g., one or more joysticks, which are configured to correspond to respective robotic units of the robotic system. For example, the system may include first and second robotic units, and the control-component unit may include first and second joysticks to be operated by the operators right and left hands. For some applications, the control-component joysticks comprise respective control-component tools therein (in order to replicate the robotic units). Typically, the computer processor determines the XYZ location and orientation of a tip of the control-component tool, and drives the robotic unit such that the tip of the actual tool that is being used to perform the procedure tracks the movements of the tip of the control-component tool.

It is typically the case that the physical form of a surgical tool is primarily dictated by the tool's purpose, and the operational constraints. Only secondarily is the operator's ease-of-use considered. For instance, a keratome blade typically has a sharp tip that is offset from its handle by a 45 degree angle bend. Although it might be more intuitive to use a straight tool, the bend is necessary because the patient's facial anatomy may obstruct such a tool. A prominent brow, for example, would not allow a straight keratome blade to incise the cornea at the correct angle. Therefore, the operator typically uses a bent tool, which may be less convenient to use. Furthermore, as described hereinabove, typically during an ophthalmic procedure, while the tip of a tool is moved within the patient's anterior capsule, these movements are constrained such as to maintain the insertion location of the tool into the anterior capsule to be via the incision region in the cornea. This constraint in the movements of the tool are typically cumbersome for the operator.

In accordance with applications of the present invention, some of the limitations described in the above paragraph are alleviated by the use of a virtual tool, as described in further detail hereinbelow with reference to a number of examples. Such virtual tools are virtual representations of surgical tools that are conveniently shaped such that they can be moved in ways that may not be possible in standard surgery. The virtual tools are displayed to an operator overlaid upon an image of the patient's eye. The virtual tools are displayed to an operator overlaid upon an image of the patient's eye and a real tool. For some applications, the virtual tools are controlled by moving tools of the control-component unit. For some applications, the robotic system is configured to facilitate switching between (a) real-tool mode, whereby the computer processor drives the tool to move in a manner that directly correspond to inputs from the operator via control-component unit (e.g., the control-component tool), and (b) virtual-tool mode, whereby the computer processor interprets inputs from the operator via the control-component unit (e.g., the control-component tool) as indicative of moving the virtual tool in a given manner, from which the computer processor derives how to move real tool in a corresponding manner.

As described hereinabove, typically, the robotic system includes an imaging system that acquires images of the portion of the patient's body that is operated on (e.g., the patient's eye) and one or more displays that display the image of the portion of the patient's body to an operator (e.g., healthcare professionals, such as a physician and/or a nurse). For some applications, one of the tools is disposed at a given orientation with respect to the portion of the patient's body (which is the orientation in which the tool is typically disposed in order to perform a stage of the procedure). A virtual tool is oriented in a different orientation with respect to the image of the portion of the patient's body (which is typically an orientation in which the operator finds it convenient to handle the tool). The operator moves a given portion of the virtual tool (the tip of the tool) with respect to the image of the portion of the patient's body (e.g., by the operator controlling movement of the virtual tool using movement of the control-component tool as an input). The computer processor typically receives an input that is indicative of the operator having moved the portion of the virtual tool with respect to the image of the portion of the patient's body. In response thereto, the computer processor drives the corresponding portion of the tool (the tip of the tool) to move with respect to the portion of the patient's body in a corresponding manner.

Typically, the computer processor drives the portion of the tool to move with respect to the portion of the patient's body in a manner that corresponds to movement of the portion of the virtual tool with respect to the portion of the patient's body, without adjusting the orientation of the tool to conform to the orientation of the virtual tool. For example, the virtual tool may be oriented substantially perpendicular to the patient's cornea within the image (or substantially perpendicular to a surface of another portion of the patient's body within the image) and the tool may be oriented at an angle with respect to the patient's cornea (or at an angle to the surface of the other portion of the patient's body). For some applications, the computer processor moves the tip of the real tool with respect to the portion of the patient's body in a manner that corresponds to movement of the tip of the virtual tool with respect to the portion of the patient's body, while maintaining an RCM of the tool within an incision region within the patient's cornea.

For some applications, in response to receiving an input indicating that the operator moves a given portion of the virtual tool (e.g., a tip of the virtual tool) in a pattern of movement (e.g., a circular pattern of movement) with respect to the image of the portion of the patient's body, the computer processor may drive a portion of the tool (e.g., the tip of the tool) to move with respect to the portion of the patient's body in the same pattern of movement (e.g., in order to make a circular incision in the patient's cornea).

There is therefore provided, in accordance with the present invention, apparatus for performing a procedure on a portion of a body of a patient, the apparatus including:
an imaging device configured to image the portion of the patient's body;
a display configured to display the image of the portion of the patient's body to an operator;
a robotic unit configured to hold a tool at a given orientation with respect to the portion of the patient's body; and
at least one computer processor configured to:
   drive the display to display a virtual tool, which is a virtual representation of the tool that is conveniently shaped such that it can be moved in ways that may not be possible in standard surgery, overlaid upon the image of the portion of the patient's body, such that at least a tip of the virtual tool coincides with a corresponding tip of the tool but an orientation of the virtual tool differs from the given orientation;
   receive an input from an operator indicating that the tip of the virtual tool should be moved in a given manner, and
   in response thereto, to drive the tool to move such that the corresponding tip of the tool moves with respect to the portion of the patient's body, correspondingly to the given manner.

In some applications, the apparatus further includes a control component, the control component is configured to be moved by the operator, and the computer processor is configured to receive movement of the control component as the input indicating that the tip of the virtual tool should be moved in the given manner.

In some applications, the computer processor is configured to:
receive another input indicating that the computer processor should control movement of the tool in a real-tool mode, and
in response thereto, drive the tool to move in a manner that directly correspond to inputs from the operator via the control component.

In some applications, a second portion of the tool is controlled by the robotic unit such as to define a remote center of motion as the corresponding portion of the tool is moved.

In some applications, a portion of the tool that is proximal to the tip of the tool is controlled by the robotic unit such as to define a remote center of motion as the tip of the tool is moved.

In some applications, the portion of the subject's body includes an eye of the subject's body, and the portion of the tool that is proximal to the tip of the tool is controlled by the robotic unit is kept within an incision region within a cornea of the subject as the tip of the tool is moved.

The present invention will be more fully understood from the following detailed description of embodiments thereof, taken together with the drawings, in which:

### BRIEF DESCRIPTION OF THE DRAWINGS

Fig. 1 is a schematic illustration of a robotic system that is configured for use in a microsurgical procedure, such as intraocular surgery;
Fig. 2 is a schematic illustration of a set of tools for mounting upon a tool mount of an end effector of a robotic unit;
Fig. 3 is a schematic illustration of a tool mounted upon a tool mount of an end effector of a robotic unit;
Fig. 4 is a schematic illustration of a robotic unit;
Figs. 5A and 5B are schematic illustrations of a keratome blade that is used for making an incision in a patient's cornea, with Fig. 5B showing a virtual keratome blade overlaid on the illustration of the keratome blade, in accordance with some applications of the present invention;
Figs. 6A and 6B are schematic illustrations of forceps that are typically inserted into a patient's eye, with Fig. 6B showing several configurations of virtual forceps overlaid on the illustration of the forceps, in accordance with some applications of the present invention;
Figs. 7A and 7B are schematic illustrations of a phacoemulsification probe and a virtual tool overlaid on the phacoemulsification probe, in accordance with some applications of the present invention; and
Fig. 8 is a schematic illustration of a display and a control-component unit of a robotic system.

### DETAILED DESCRIPTION OF EMBODIMENTS

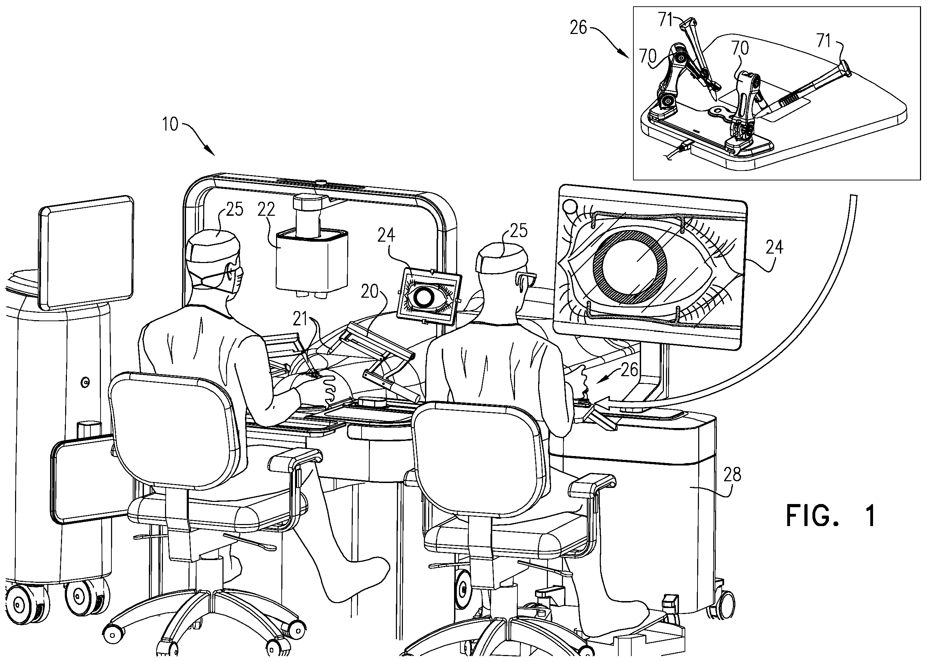

Reference is now made to Fig. 1, which is a schematic illustration of a robotic system 10 that is configured for use in a microsurgical procedure, such as intraocular surgery. Typically, when used for intraocular surgery, robotic system 10 includes one or more robotic units 20 (which are configured to hold tools 21), in addition to an imaging system 22 (e.g., one or more imaging devices, such as cameras), one or more displays 24 and a control-component unit 26 (for example, a control-component unit that includes a pair of control components, such as joysticks 70, as shown in the enlarged portion of Fig. 1), via which one or more operators 25 (e.g., healthcare professionals, such as a physician and/or a nurse) is able to control robotic units 20. Typically, robotic system 10 includes one or more computer processors 28, via which components of the system and operator(s) 25 operatively interact with each other. The scope of the present application includes mounting one or more robotic units in any of a variety of different positions with respect to each other.

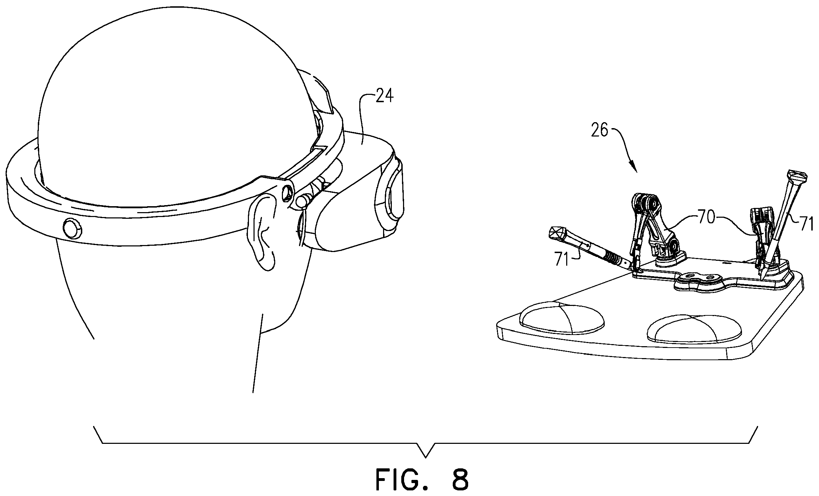

Typically, movement of the robotic units (and/or control of other aspects of the robotic system) is at least partially controlled by one or more operators 25 (e.g., healthcare professionals, such as a physician and/or a nurse). For example, the operator may receive images of the patient's eye and the robotic units, and/or tools disposed therein, via display 24. Typically, such images are acquired by imaging system 22. Typically, the imaging system includes one or more cameras and/or one or more microscopes. For some applications, imaging system 22 is a stereoscopic imaging device and display 24 is a stereoscopic display. For some applications, display 24 is a head-mounted display (e.g., a head-mounted stereoscopic display), as shown in Fig. 8. Based on the received images, the operator typically performs steps of the procedure. For some applications, the operator provides commands to the robotic units via control-component unit 26. Typically, such commands include commands that control the position and/or orientation of tools that are disposed within the robotic units, and/or commands that control actions that are performed by the tools. For example, the commands may control a blade, a phacoemulsification tool (e.g., the operation mode and/or suction power of the phacoemulsification tool), and/or injector tools (e.g., which fluid (e.g., viscoelastic fluid, saline, etc.) should be injected, and/or at what flow rate). Alternatively or additionally, the operator may input commands that control the imaging system (e.g., the zoom, focus, and/or x-y positioning of the imaging system). For some applications, the commands include controlling an intraocular-lens-manipulator tool, for example, such that the tool manipulates the intraocular lens inside the eye for precise positioning of the intraocular lens within the eye.

Typically, the control-component unit includes one or more control components, e.g., one or more joysticks 70 that are configured to correspond to respective robotic units 20 of the robotic system. For example, as shown, the system may include first and second robotic units, and the control-component unit may include first and second joysticks, as shown. For some applications, the control-component joysticks comprise respective control-component tools 71 therein (in order to replicate the robotic units), as shown in Fig. 1. Typically, the computer processor determines the XYZ location and orientation of the tip of the control-component tool 71, and drives the robotic unit such that the tip of the actual tool 21 that is being used to perform the procedure tracks the movements of the tip of the control-component tool. For some applications, the control-component tools 71 are used in a similar manner to that described above, but with the control-component tools 71 being oriented to correspond to a virtual tool, as described in further detail hereinbelow.

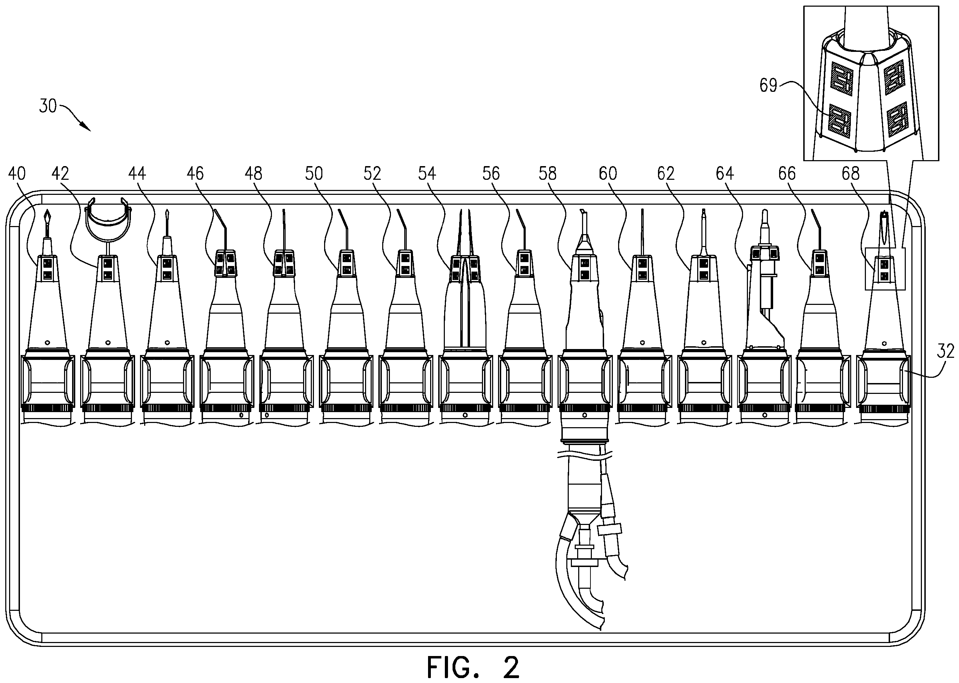

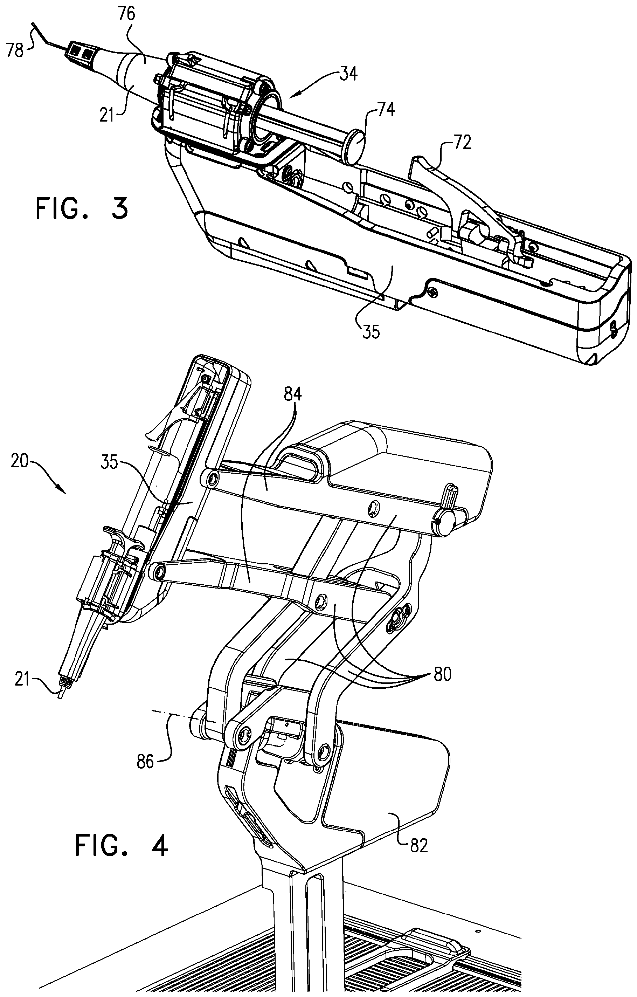

Reference is now made to Fig. 2, which is a schematic illustration of a set 30 of tools that are configured to be mounted upon a tool mount 34 of an end effector 35 (tool mount and end effector being shown in Fig. 3) of robotic unit 20. For some applications, the tools are those that are typically used in a cataract procedure, a different ophthalmic procedure, and/or a different microsurgical procedure. For example, as shown in Fig. 2, the tools typically include one or more of the following tools: a keratome blade 40, an eye fixator 42, a paracentesis knife 44, a dispersive ophthalmic viscosurgical device (OVD) syringe 46, a cohesive ophthalmic viscosurgical device (OVD) syringe 48, a staining syringe 50 (e.g., for staining the anterior lens with a stain such as trypan blue ophthalmic solution), a lidocaine syringe 52, forceps 54, a hydrodissection syringe 56, a phacoemulsification probe 58, a chopper 60, an irrigation/aspiration probe 62, an intraocular lens injector 64, an antibiotics syringe 66, and/or a Limbal Relaxing Incision (LRI) knife 68. For some applications, each of the tools includes one or more markers 69, which may be used to identify the tool and/or to determine the position and/or orientation of the tool.

Reference is now made to Fig. 3, which is a schematic illustration of tool 21 mounted upon tool mount 34 of end effector 35 of robotic unit 20. Tool mount 34 is typically coupled to, or formed integrally with, end effector 35 of the robotic unit. Robotic unit 20 is typically configured to drive the tool to roll with respect to the tool mount, for example, by driving a gear wheel (not shown) of the end effector to roll a gear wheel (not shown) of the tool, to thereby cause the tool to roll. For some applications, tool 21 is configured to be actuated to perform a function via a linear tool-actuation arm 72, which is disposed on end effector 35 and is configured to push a portion of the tool axially. Typically, the linear tool-actuation arm pushes the portion of the tool distally with respect to the tool mount and the mount-engagement portion (i.e., such that the portion of the tool distally moves distally relative to the tool mount and the mount-engagement portion. For example, as shown in Fig. 3, the tool 21 that is placed within the tool mount is a syringe (e.g., dispersive ophthalmic viscosurgical device (OVD) syringe 46, cohesive ophthalmic viscosurgical device (OVD) syringe 48, staining syringe 50, lidocaine syringe 52, hydrodissection syringe 56, intraocular lens injector 64, and/or antibiotics syringe 66). Typically, the syringe includes a plunger 74, a barrel 76, and a cannula 78. In such cases, the linear tool-actuation arm is configured to push plunger 74 of the syringe axially in a forward direction. For some applications, a portion of the tool is configured to be moved with respect to the patient's eye by linear tool-actuation arm 72 pushing a portion of the tool axially.

Reference is now made to Fig. 4, which is schematic illustration of robotic unit 20. Robotic unit 20 typically includes one or more robotic arms 80 that are coupled to end effector 35 and that are disposed on a robotic-unit base 82. For some applications, the robotic arms are configured to rotate tools that are coupled to the end effector through pitch and yaw rotations by the one or more robotic arms moving. Typically, the robotic unit is configured to insert tools 21 into the patient's eye such that entry of the tool into the patient's eye is via an incision point, and the tip of the tool is disposed within the patient's eye. Further typically, the robotic unit is configured to move the tip of the tool within the patient's eye in such a manner that entry of the tool into the patient's eye is constrained to remain within the incision point. For some applications, the robotic unit achieves this by being configured such as to define a remote center of motion ("RCM") of the tool and the RCM of the tool is made to coincide with the incision point. It is noted that, for some applications, the robotic unit is configured to move the tip of the tool within the patient's eye in such a manner that entry of the tool into the patient's eye is constrained to remain within an incision zone, rather than an incision point, as described in further detail hereinbelow. For such cases, all aspects of the present application that are described as being applied to an incision point are applied to an incision zone, *mutatis mutandis.* The term "incision region" is used herein to denote either an incision point or an incision zone.

Typically, the robotic arms include a parallelogram structure that includes one or more pairs of parallel arms, with one pair 84 of parallel arms disposed one above the other (at least when the robotic arms are disposed in a central orientation with respect to yaw rotation of the robotic unit). Further typically, the parallel arms function to constrain movement of the end effector, and thereby constrain the motion of tool 21, such that as the tool undergoes changes in pitch, the RCM of the tool is maintained. For some applications, the robotic arms are configured to rotate tools that are coupled to the end effector through yaw rotation by rotating about an axis 86. Typically, this results in the tool rotating about a virtual axis (which is the extrapolation of axis 86). Further typically, the rotation of the tool about the virtual axis is such that as the tool undergoes changes in yaw angular position, the RCM of the tool is maintained.

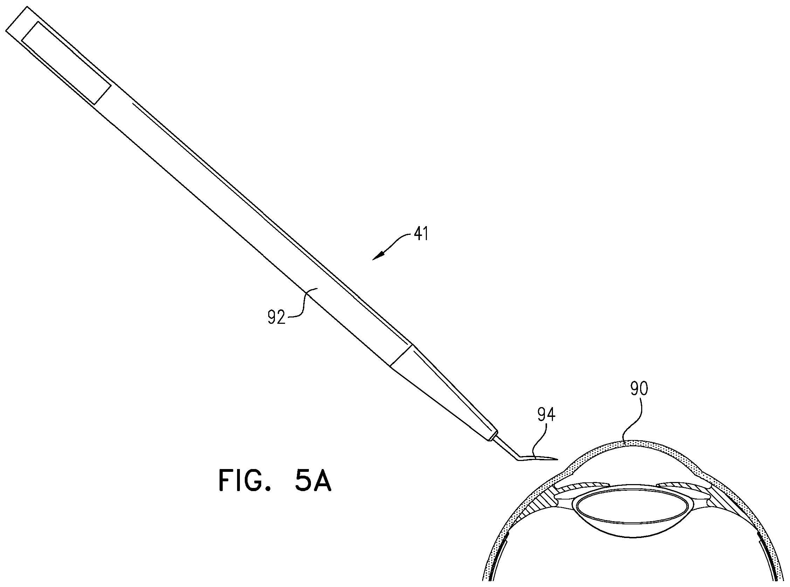

Reference is now made to Fig. 5A, which is a schematic illustration of a keratome blade 40 that is used for making an incision in a patient's cornea 90. It is typically the case that the physical form of a surgical tool is primarily dictated by the tool's purpose, and the operational constraints. Only secondarily is the operator's ease-of-use considered. For instance, as shown, a keratome blade typically has a sharp tip 94 that is offset from its handle 92 by a 45 degree angle bend. Although it might be more intuitive to use a straight tool, the bend is necessary because the patient's facial anatomy may obstruct such a tool. A prominent brow, for example, would not allow a straight keratome blade to incise the cornea at the correct angle. Therefore, the operator typically uses a bent tool, which may be less convenient to use. Furthermore, as described hereinabove, typically during an ophthalmic procedure, while the tip of a tool is moved within the patient's anterior capsule, these movements are constrained such as to maintain the insertion location of the tool into the anterior capsule to be via the incision region in the cornea. This constraint in the movements of the tool are typically cumbersome for the operator.

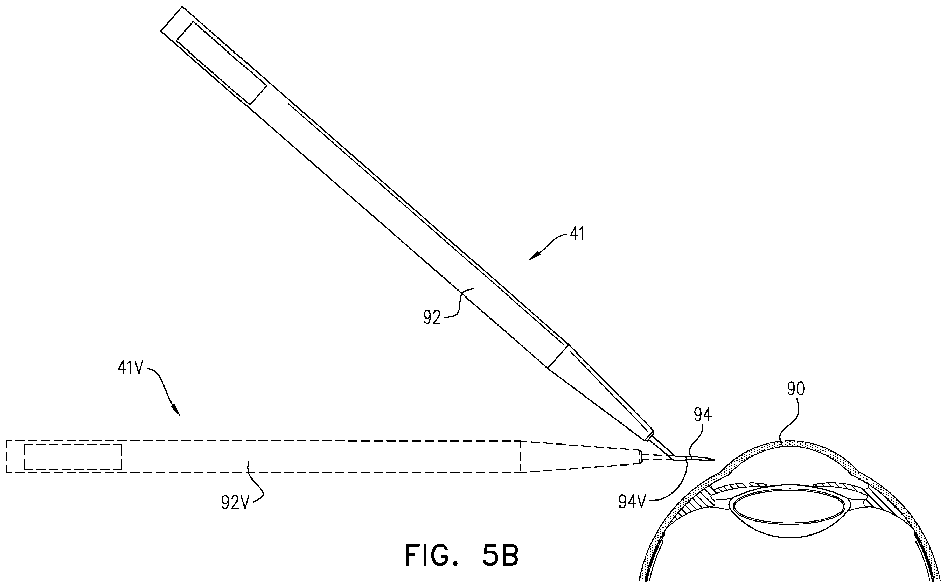

In accordance with some applications of the present invention, some of the limitations described in the above paragraph are alleviated by the use of a virtual tool, as described in further detail hereinbelow with reference to a number of examples. Such virtual tools are virtual representations of surgical tools that are conveniently shaped such that they can be moved in ways that may not be possible in standard surgery. The virtual tools are displayed to an operator overlaid upon an image of the patient's eye. The virtual tools are displayed to an operator overlaid upon an image of the patient's eye and a real tool (e.g., as illustrated schematically in each of Figs. 5B, 6B, and 7B). For some applications, the virtual tools are controlled by moving tools 71 of control-component unit 26. For some applications, robotic system 10 is configured to facilitate switching between (a) real-tool mode, whereby the computer processor drives the tool to move in a manner that directly correspond to inputs from the operator via control-component unit 26 (e.g., control-component tool 71), and (b) virtual-tool mode, whereby the computer processor interprets inputs from the operator via control-component unit 26 (e.g., control-component tool 71) as indicative of moving the virtual tool in a given manner, from which the computer processor derives how to move real tool in a corresponding manner, as described in further detail hereinbelow with reference to Fig. 8.

As described hereinabove, robotic system 10 includes an imaging system that acquires images of the portion of the patient's body that is operated on (e.g., the patient's eye) and one or more displays that display the image of the portion of the patient's body to an operator (e.g., healthcare professionals, such as a physician and/or a nurse). One of tools 21 is disposed at a given orientation with respect to the portion of the patient's body (which is the orientation in which the tool is typically disposed in order to perform a stage of the procedure). A virtual tool is oriented in a different orientation with respect to the image of the portion of the patient's body (which is typically an orientation in which the operator finds it convenient to handle the tool). The operator moves a given portion of the virtual tool (the tip of the tool) with respect to the image of the portion of the patient's body (e.g., by the operator controlling movement of the virtual tool using movement of control-component tool 71 as an input). Computer processor 28 typically receives an input that is indicative of the operator having moved the portion of the virtual tool with respect to the image of the portion of the patient's body. In response thereto, the computer processor drives the corresponding portion of the tool (the tip of the tool) to move with respect to the portion of the patient's body in a corresponding manner.

Typically, the computer processor drives the portion of the tool to move with respect to the portion of the patient's body in a manner that corresponds to movement of the portion of the virtual tool with respect to the portion of the patient's body, without adjusting the orientation of the tool to conform to the orientation of the virtual tool. For example, the virtual tool may be oriented substantially perpendicular to the patient's cornea within the image (or substantially perpendicular to a surface of another portion of the patient's body within the image) and the tool may be oriented at an angle with respect to the patient's cornea (or at an angle to the surface of the other portion of the patient's body). For some applications, the computer processor moves the tip of the real tool with respect to the portion of the patient's body in a manner that corresponds to movement of the tip of the virtual tool with respect to the portion of the patient's body, while maintaining an RCM of the tool within an incision region within the patient's cornea.

For some applications, in response to receiving an input indicating that the operator moves a given portion of the virtual tool (a tip of the virtual tool) in a pattern of movement (e.g., a circular pattern of movement) with respect to the image of the portion of the patient's body, the computer processor may drive a portion of the tool (the tip of the tool) to move with respect to the portion of the patient's body in the same pattern of movement (e.g., in order to make a circular incision in the patient's cornea).

Some examples of the use of a virtual tool are described in further detail hereinbelow with reference to Figs. 5B-7B.

As noted above with reference to Fig. 5A, a keratome blade 41 typically has a sharp tip 94 that is offset from its handle 92 by a 45 degree angle bend. (Keratome blade 41 as shown in Fig. 5A is designed differently from keratome blade 40 shown in Fig. 2, by way of illustration. The scope of the present disclosure includes applying the techniques described herein to any type of keratome blade or any tool, *mutatis mutandis.*) For some applications, the ophthalmic operator uses a virtual keratome 41V having a straight tip that is aligned with its handle 92V, as shown in Fig. 5B. The operator moves the tip of the virtual tool with respect to an image of the patient's eye. Computer processor 28 drives tip 94 of keratome blade 41 to move with respect to the patient's eye in a corresponding manner. For example, when the virtual keratome blade is translated or rotated, the real keratome blade is translated or rotated accordingly. Typically, this allows the operator to perform a corneal incision by moving the virtual tool along the axis of its handle, without having to account for bend in its tip. Fig. 5B schematically illustrates how movement of the virtual keratome blade by the operator would correspond movement of keratome blade 41.

It is noted that the techniques described with reference to Figs. 5A-B may be practiced with any one of the tools described with reference to Fig. 2, *mutatis mutandis.* The example of keratome blade 41 that is provided in Figs. 5A and 5B is merely illustrative.

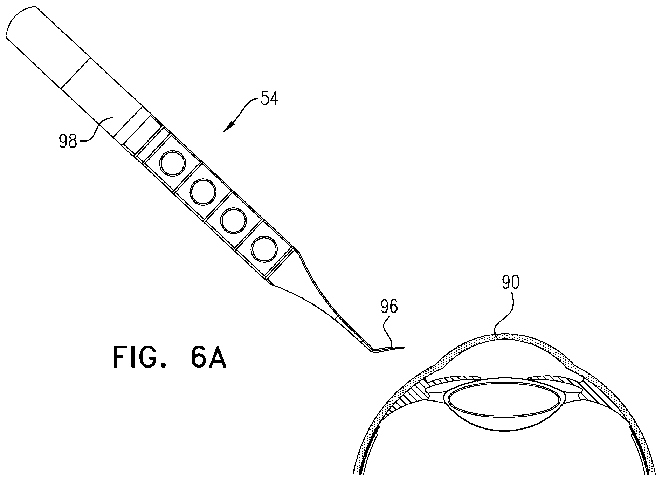

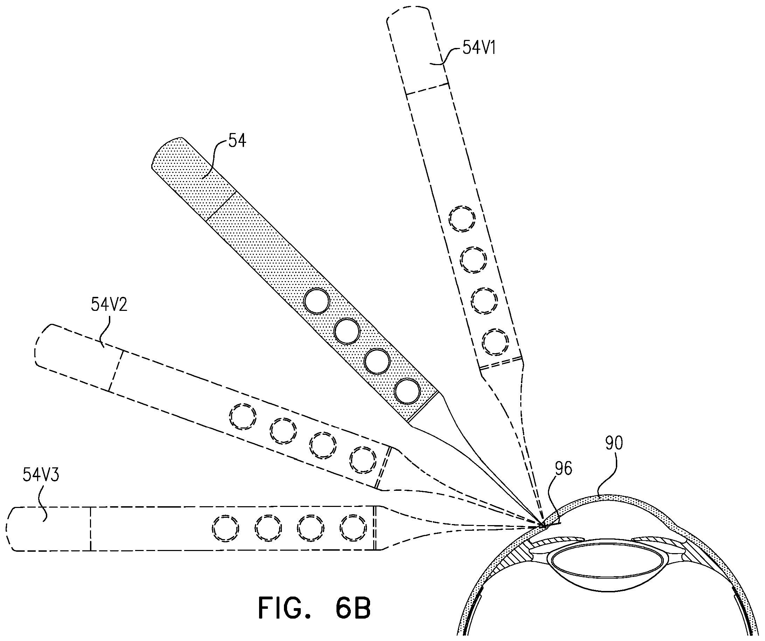

Reference is now made to Figs. 6A and 6B, which are schematic illustrations of forceps 54 that are typically inserted into a patient's eye, with Fig. 6B showing several configurations of virtual forceps 54V1, 54V2, 54V3 overlaid on the illustration of the forceps, in accordance with some applications of the present invention. Fig. 6B schematically illustrates how movement of the virtual forceps by the operator would correspond movement of forceps 54. In accordance with some applications, using virtual tools, the operator may decide to modify an existing tool, e.g., by changing the form of the tool except for the tip. With respect to the example of forceps, which are shown, the tip 96 of ophthalmic forceps are typically bent at approximately a 45 degree angle to the handle 98, for similar reasons to those provided hereinabove with respect to the keratome blade. An operator might prefer to handle forceps having a tip that is disposed at a different angle, or forceps having different dimensions (e.g., shorter or longer forceps). For some applications, the operator is able to do so, by using virtual forceps.

Referring to Fig. 6B, virtual forceps 54V1 are configured to be oriented at an orientation that is perpendicular (or approximately perpendicular) to the surface of the cornea at the center of the cornea. In some cases, an operator may find it more intuitive or comfortable to move the tip of forceps that are oriented in this orientation as compared with forceps 54. However, it is not possible to do this with real forceps because they cannot be safely inserted through the cornea from this orientation. Virtual forceps 54V2 and 54V3 have tips that are disposed at a smaller angle from their respective handles than tip 96 of forceps 54. In some cases, an operator may find it more intuitive or comfortable to move the tip of forceps that are oriented in this orientation as compared with forceps 54. However, for similar reasons to those provided hereinabove with respect to the keratome blade, this is typically not possible with real forceps.

Typically, the use of virtual tools as described herein facilitates the provision of a large variety of tools being offered to the operator, with each of the variety of tools corresponding to a given real tool. For some applications, the operator selects their preferable tool parameters ahead of surgery, selects duplicate tools with different characteristics (such that (s)he has a choice of tools), and/or adjusts the tool characteristics mid-surgery, according to their convenience. For example, with reference to the example shown in Fig. 6B, the operator could select to use any one the virtual forceps, or to use the real forceps, or switch between these options either before or during the procedure.

It is noted that the techniques described with reference to Figs. 6A-B may be practiced with any one of the tools described with reference to Fig. 2, *mutatis mutandis.* The example of forceps 54 that is provided in Figs. 6A and 6B is merely illustrative.

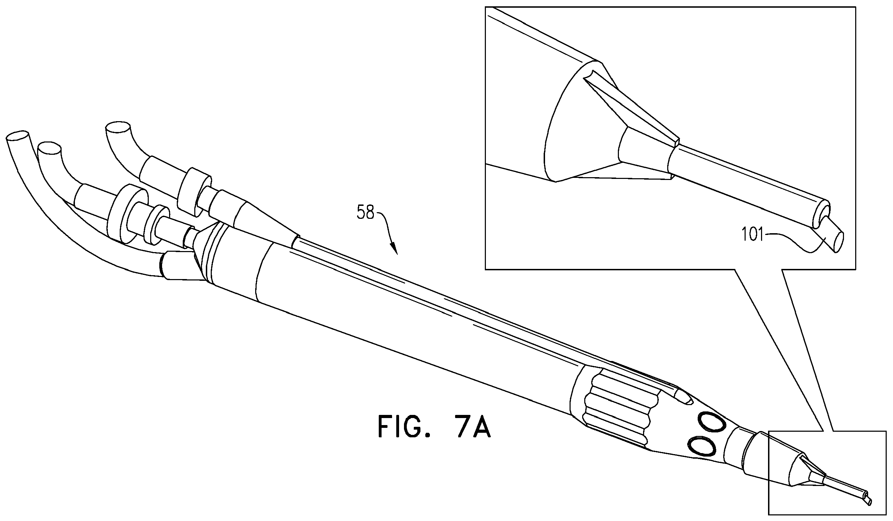

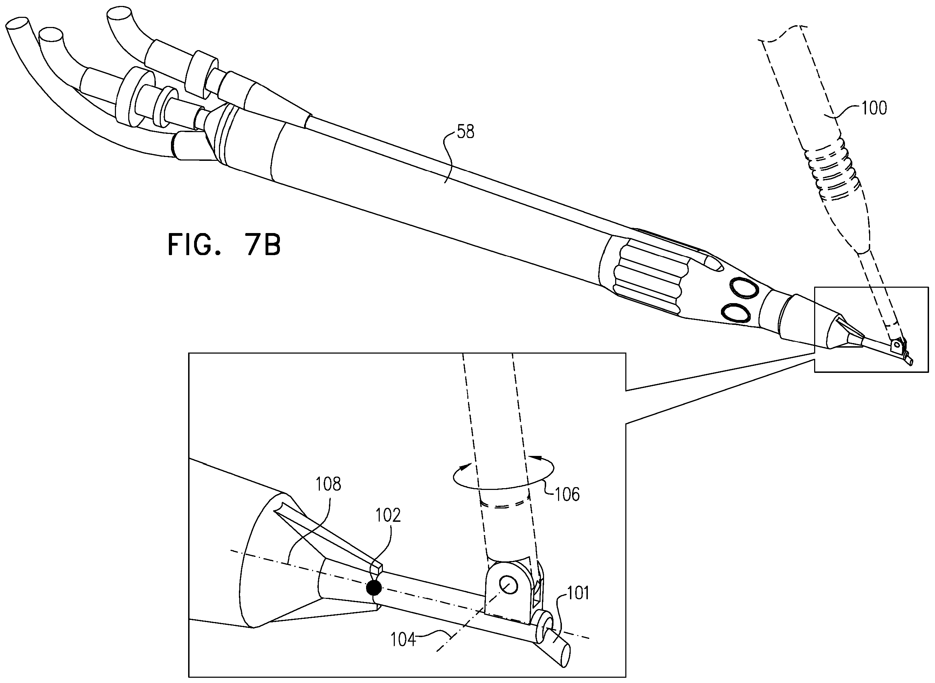

Reference is now made to Figs. 7A and 7B, which are schematic illustrations of phacoemulsification probe 58 and a virtual tool 100 overlaid on the phacoemulsification probe, in accordance with some applications of the present invention. Fig. 7A includes an enlargement of a tip 101 of the phacoemulsification probe. As described hereinabove, typically, robotic unit 20 is configured to insert tools 21 into the patient's eye such that entry of the tool into the patient's eye is via an incision region, and the tip of the tool is disposed within the patient's eye. Further typically, the robotic unit is configured to move the tip of the tool within the patient's eye in such a manner that entry of the tool into the patient's eye is constrained to remain within the incision region. For some applications, the robotic unit achieves this by being configured such as to define a remote center of motion ("RCM") of the tool and the RCM of the tool is made to coincide with the incision region. An example of this is shown in Fig. 7B, in which an RCM 102 of the phacoemulsification probe is indicated.

When robotic system 10 is moving a tool with RCM constraints in place, the tool axis typically passes through an incision region. When the tool axis is forced through an incision region, the degrees of freedom of the tool tip are limited to three angular rotations (e.g., roll, pitch, yaw) and one translation (distance of the tool tip from the incision region). For some applications, virtual tool 100 coincides with tip 101 of the phacoemulsification probe, as shown in Fig. 7B. Typically, the operator moves tip 101 using the virtual tool by moving the virtual tip through 3 translations (X, Y, and Z). Computer processor 28 drives tip 101 of phacoemulsification probe 58 to move with respect to the patient's eye in a corresponding manner, while operating under the constraint of maintaining the RCM of the phacoemulsification probe 58 within the incision region.

For some applications, the virtual tool has two rotational degrees of freedom, which do not generate movement of the phacoemulsification probe. For example, as shown, the virtual tip can be rotated about axis 104 and can roll about its own axis (as indicated by arrow 106), with these movement not generating any movement of the phacoemulsification probe. Typically, in response to rotation of the virtual tool about axis 108 of the phacoemulsification probe the computer processor drives the phacoemulsification probe to move in a corresponding manner (i.e., it causes the phacoemulsification probe to roll about its own axis in a corresponding manner).

For some applications, computer processor 28 converts movements of operator to scaled down movements of the tools 21, such that the operator is able to move the tools through smaller movements that they might otherwise be able to do. Typically, it is more intuitive to the operator to scale translation than to scale rotation. For example, an operator may readily adapt to a 1:2 translation scale, where a 2 cm movement of their hands results in a 1 cm translation of the tool. However, it is typically less intuitive to scale rotation; i.e. a 90 degree rotation of the hand scaled down to a 45 degree rotation of the tool. Therefore, for some applications virtual tools make greater use of translation (which is readily scalable), and less use of rotation (which is less readily scalable). The example provided in Figs. 7A-B is one example of this. As noted, the degrees of freedom of tip 101 of the phacoemulsification probe are limited to three angular rotations (e.g., roll, pitch, yaw) and one translation (distance of the tool tip from the incision region). However, the operator moves tip 101 using the virtual tool by moving the virtual tip through 3 translations (X, Y, and Z) and only one rotation (e.g., about axis 108 of the phacoemulsification probe).

It is noted that the techniques described with reference to Figs. 7A-B may be practiced with any one of the tools described with reference to Fig. 2, *mutatis mutandis.* The example of phacoemulsification probe 58 that is provided in Figs. 6A and 6B is merely illustrative.

Reference is now made to Fig. 8, which is a schematic illustration of display 24 and control-component unit 26, which includes control-component tools 71. As described hereinabove, for some applications, the virtual tools described herein are non-physical. For example, the virtual tools may be virtual representations of a tool. For some applications, the virtual tools are displayed to an operator overlaid upon an image of the patient's eye. For some applications, the virtual tools are displayed to an operator overlaid upon an image of the patient's eye and a real tool (e.g., as shown in each of Figs. 5B, 6B, and 7B). For some applications, the virtual tools are displayed on display 24. As noted hereinabove, for some applications, imaging system 22 is a stereoscopic imaging device and display 24 is a stereoscopic display. For some applications, the display is a head-mounted display (e.g., a stereoscopic head-mounted display), as shown.

For some applications, the virtual tools are virtual representations of a tool that are controlled by moving tools 71 of control-component unit 26. For some applications, robotic system 10 is configured to facilitate switching between (a) real-tool mode, whereby the computer processor drives the tool to move in a manner that directly correspond to inputs from the operator via control-component unit 26 (e.g., control-component tool 71), and (b) virtual-tool mode, whereby the computer processor interprets inputs from the operator via control-component unit 26 (e.g., control-component tool 71) as indicative of moving the virtual tool in a given manner, from which the computer processor derives how to move real tool in a corresponding manner.

For some such applications, the virtual tools described herein are used in combination with real tools using one or more of the following procedural steps. It is noted that some of the following steps are optional, and this sequence of steps as merely illustrative of some functions of robotic system 10, in accordance with some applications of the present disclosure.
1. The operator manipulates the real tool to a desired location and orientation with respect to the patient's eye.
2. The operator provides an input indicating that they would like to switch to using a virtual tool. For example, the input can be provided to computer processor 28 via a button, gesture, voice command, though a surgical assistant's computer interface, or any other input device or method.
3. The real tool stays in place. A representation of the virtual tool appears at the same location and orientation as the real tool. Typically, the representation of the virtual tool is a 3D graphical representation of the virtual tool. A second representation of the virtual tool also appears with the second representation being disposed at the same location and orientation as control-component tool 71.
4. The second representation of the virtual tool is moved by the operator moving the control-component tool 71. At this stage, the real tool and first representation of the virtual tool remain stationary.
5. The operator moves the second representation of the virtual tool such that at least portions of the virtual tools and the real tool that are configured to coincide (e.g., the tool tips) coincide (within a predefined tolerance of position and orientation). For some applications, the computer processor interprets this to automatically switch the robotic system to being controlled in virtual-tool mode, whereby movement of the virtual tool by the operator (using control-component tool 71), is used as an input by the computer processor to move the real tool in a corresponding manner. For some applications, in order to indicate to the operator that this has happened, an output is generated. For example, one or more of the following outputs may be generated:
   a. The second representation of the virtual tool disappears.
   b. The first representation of the virtual tool changes e.g., its opacity increases.
   c. The real tool is blurred, made to be partially transparent, or removed digitally from the display.
   d. The operator is given an indication that the switch has been made. This indication can be haptic feedback through the manipulation apparatus, a display indication, a flashing of the virtual tool color/brightness, an aural indicator, or any other indicator or combination of the aforementioned indicators.
   For some applications, the computer processor switches the robotic system to being controlled in virtual tool mode in response to an input from the operator (as an alternative or in addition to automatically switching the robotic system to being controlled in virtual tool mode).
6. The operator continues to operate, using the virtual tool to guide the real tool.

For some applications, in order to switch from virtual-tool mode, back to real-tool mode, similar steps to steps 1-6 above are applied, but the virtual tool representation is moved such as to coincide with the real tool.

For some applications, there are certain tools that are predefined to always be operated in virtual tool mode by default, such that, by default there is no need to switch to virtual-tool mode, as described above.

It is noted that in the context of the present application, any description of portions of real and virtual tools coinciding should be interpreted to mean that at least some of the portion of the virtual tool (e.g., a tip region of the virtual tool) and at least some of the portion of the real tool (e.g., a tip region of the real tool) coincide within a predefined tolerance of position and orientation. It should not be interpreted to mean that there is precise coincidence of the entire portion of the virtual tool (e.g., the entire virtual tool tip) with the entire portion of the real tool (e.g., the entire real tool tip).

Although some applications of the present disclosure are described with reference to cataract surgery, the scope of the present application includes applying the apparatus described herein to other medical procedures, *mutatis mutandis.* In particular, the apparatus and methods described herein to other medical procedures may be applied to other microsurgical procedures, such as general surgery, orthopedic surgery, gynecological surgery, otolaryngology, neurosurgery, oral and maxillofacial surgery, plastic surgery, podiatric surgery, vascular surgery, and/or pediatric surgery that is performed using microsurgical techniques. For some such applications, the imaging system includes one or more microscopic imaging units.

It is noted that the scope of the present application includes applying the apparatus described herein to intraocular procedures, other than cataract surgery, *mutatis mutandis.* Such procedures may include collagen crosslinking, endothelial keratoplasty (e.g., DSEK, DMEK, and/or PDEK), DSO (descemet stripping without transplantation), laser assisted keratoplasty, keratoplasty, LASIK/PRK, SMILE, pterygium, ocular surface cancer treatment, secondary IOL placement (sutured, transconjunctival, etc.), iris repair, IOL reposition, IOL exchange, superficial keratectomy, Minimally Invasive Glaucoma Surgery (MIGS), limbal stem cell transplantation, astigmatic keratotomy, Limbal Relaxing Incisions (LRI), amniotic membrane transplantation (AMT), glaucoma surgery (e.g., trabs, tubes, minimally invasive glaucoma surgery), automated lamellar keratoplasty (ALK), anterior vitrectomy, and/or pars plana anterior vitrectomy.

Applications of the invention described herein can take the form of a computer program product accessible from a computer-usable or computer-readable medium (e.g., a non-transitory computer-readable medium) providing program code for use by or in connection with a computer or any instruction execution system, such as computer processor 28. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W), DVD, and a USB drive.

A data processing system suitable for storing and/or executing program code will include at least one processor (e.g., computer processor 28) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the algorithms described herein, can be implemented by computer program instructions. These computer program instructions may be provided to a processor of a general-purpose computer, special purpose computer, or other programmable data processing apparatus to produce a machine, such that the instructions, which execute via the processor of the computer (e.g., computer processor 28) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

Computer processor 28 is typically a hardware device programmed with computer program instructions to produce a special purpose computer. For example, when programmed to perform the algorithms described with reference to the Figures, computer processor 28 typically acts as a special purpose robotic-system computer processor. Typically, the operations described herein that are performed by computer processor 28 transform the physical state of a memory, which is a real physical article, to have a different magnetic polarity, electrical charge, or the like depending on the technology of the memory that is used. For some applications, operations that are described as being performed by a computer processor are performed by a plurality of computer processors in combination with each other.

It will be appreciated by persons skilled in the art that the present invention is not limited to what has been particularly shown and described hereinabove. Rather, the scope of the present invention is defined by the appended claims.

## Claims

1. Apparatus (10) for performing a procedure on a portion of a body of a patient, the apparatus comprising:
an imaging device (22) configured to image the portion of the patient's body;
a display (24) configured to display the image of the portion of the patient's body to an operator (25);
a robotic unit (20) configured to hold a tool (21) at a given orientation with respect to the portion of the patient's body; and
at least one computer processor (28) configured to:
drive the display to display a virtual tool (100), which is a virtual representation of the tool that is conveniently shaped such that it can be moved in ways that may not be possible in standard surgery, overlaid upon the image of the portion of the patient's body, such that at least a tip of the virtual tool coincides with a corresponding tip of the tool but an orientation of the virtual tool differs from the given orientation,
receive an input from the operator indicating that the tip of the virtual tool should be moved in a given manner, and
in response thereto, drive the robotic unit to move the tool such that the corresponding tip of the tool moves, with respect to the portion of the patient's body, correspondingly to the given manner.

2. The apparatus according to claim 1, wherein the input indicates that the tip of the virtual tool should be moved in a circular pattern of movement with respect to a surface of the portion of the patient's body within the image, and wherein the computer processor is configured to drive the robotic unit to move the tip of the tool in the circular pattern of movement.

3. The apparatus according to claim 1, further comprising a control component, wherein the control component is configured to be moved by the operator, and wherein the computer processor is configured to receive movement of the control component as the input indicating that the tip of the virtual tool should be moved in the given manner.

4. The apparatus according to claim 3, wherein the control component comprises a joystick comprising a control-component tool oriented to correspond to the virtual tool.

5. The apparatus according to any one of claims 3-4, wherein the computer processor is configured to:
receive another input indicating that the computer processor should control movement of the tool in a real-tool mode, and
in response thereto, drive the robotic unit to move the tool in a manner that directly corresponds to control inputs from the operator via the control component.

6. The apparatus according to claim 5, wherein the computer processor is configured to switch between the real-tool mode and a virtual-tool mode, in which the computer processor interprets the control inputs as indicative of moving the virtual tool.

7. The apparatus according to claim 6, wherein the computer processor is configured to, upon switching to the virtual-tool mode, perform an operation selected from the group of operations consisting of: blurring the tool on the display, making the tool partially transparent on the display, and digitally removing the tool from the display.

8. The apparatus according to any one of claims 1 and 3-7, wherein the computer processor is configured to maintain a remote center of motion of the tool as the corresponding tip of the tool is moved.

9. The apparatus according to claim 8, wherein the computer processor is configured to maintain the remote center of motion by driving the robotic unit to control a portion of the tool, which is proximal to the tip of the tool, such as to define the remote center of motion as the tip of the tool is moved.

10. The apparatus according to claim 9, wherein the portion of the patient's body includes an eye of the patient's body, and wherein the computer processor is configured to drive the robotic unit to keep the portion of the tool within an incision region within a cornea of the patient as the tip of the tool is moved.

11. The apparatus according to any one of claims 1-9, wherein the computer processor is configured to drive the robotic unit to move the tool without adjusting an orientation of the tool to conform to the orientation of the virtual tool.

12. The apparatus according to any one of claims 1, 3-9, and 11, wherein:
the virtual tool is oriented substantially perpendicular to a surface of the portion of the patient's body within the image, and
the tool is oriented at an angle with respect to the surface of the patient's body.

13. The apparatus according to claim 12, wherein:
the portion of the patient's body includes an eye of the patient,
the virtual tool is oriented substantially perpendicular to a center of a cornea of the patient's eye within the image, and
the tool is oriented at the angle with respect to the cornea of the patient's eye.

14. A computer software product comprising a tangible non-transitory computer-readable medium in which program instructions are stored, which instructions, when read by a computer processor (28), cause the computer processor to:
drive a display (24) to display a virtual tool (100), which is a virtual representation of a tool (21) that is held by a robotic unit (20) at a given orientation with respect to a portion of a body of a patient, overlaid upon an image of the portion of the patient's body, the virtual representation being conveniently shaped such that it can be moved in ways that may not be possible in standard surgery, such that at least a tip of the virtual tool coincides with a corresponding tip of the tool but an orientation of the virtual tool differs from the given orientation,
receive an input from an operator (25) indicating that the tip of the virtual tool should be moved in a given manner, and
in response to receiving the input, drive the robotic unit to move the tool such that the corresponding tip of the tool moves, with respect to the portion of the patient's body, correspondingly to the given manner.

## Patentansprüche

1. Einrichtung (10) zum Durchführen eines Eingriffs an einem Teil eines Körpers eines Patienten, wobei die Einrichtung Folgendes umfasst:
eine Abbildungsvorrichtung (22), die dazu konfiguriert ist, den Teil des Körpers des Patienten abzubilden;
eine Anzeige (24), die dazu konfiguriert ist, das Bild des Teils des Körpers des Patienten einem Bediener (25) anzuzeigen;
eine Robotereinheit (20), die dazu konfiguriert ist, ein Werkzeug (21) in einer gegebenen Ausrichtung in Bezug auf den Teil des Körpers des Patienten zu halten; und
zumindest einen Computerprozessor (28), der zu Folgendem konfiguriert ist:
Antreiben der Anzeige, um ein virtuelles Werkzeug (100) anzuzeigen, das eine virtuelle Darstellung des Werkzeugs ist, das zweckmäßigerweise derart geformt ist, dass es auf Weisen bewegt werden kann, die bei Standardchirurgie möglicherweise nicht möglich sind, überlagert auf dem Bild des Teils des Körpers des Patienten, sodass zumindest eine Spitze des virtuellen Werkzeugs mit einer entsprechenden Spitze des Werkzeugs zusammenfällt, aber sich eine Ausrichtung des virtuellen Werkzeugs von der gegebenen Ausrichtung unterscheidet,
Empfangen einer Eingabe von dem Bediener, die angibt, dass die Spitze des virtuellen Werkzeugs auf eine gegebene Weise bewegt werden sollte, und
als Reaktion darauf Antreiben der Robotereinheit, um das Werkzeug zu bewegen, sodass sich die entsprechende Spitze des Werkzeugs in Bezug auf den Teil des Körpers des Patienten entsprechend der gegebenen Weise bewegt.

2. Einrichtung nach Anspruch 1, wobei die Eingabe angibt, dass die Spitze des virtuellen Werkzeugs in einem kreisförmigen Bewegungsmuster in Bezug auf eine Oberfläche des Teils des Körpers des Patienten innerhalb des Bildes bewegt werden sollte, und wobei der Computerprozessor dazu konfiguriert ist, die Robotereinheit anzutreiben, um die Spitze des Werkzeugs in dem kreisförmigen Bewegungsmuster zu bewegen.

3. Einrichtung nach Anspruch 1, ferner umfassend eine Steuerkomponente, wobei die Steuerkomponente dazu konfiguriert ist, durch den Bediener bewegt zu werden, und wobei der Computerprozessor dazu konfiguriert ist, Bewegung der Steuerkomponente als die Eingabe zu empfangen, die angibt, dass die Spitze des virtuellen Werkzeugs in der gegebenen Weise bewegt werden sollte.

4. Einrichtung nach Anspruch 3, wobei die Steuerkomponente einen Joystick umfasst, der ein Steuerkomponentenwerkzeug umfasst, das dazu ausgerichtet ist, dem virtuellen Werkzeug zu entsprechen.

5. Einrichtung nach einem der Ansprüche 3-4, wobei der Computerprozessor zu Folgendem konfiguriert ist:
Empfangen einer anderen Eingabe, die angibt, dass der Computerprozessor Bewegung des Werkzeugs in einem realen Werkzeugmodus steuern sollte, und
als Reaktion darauf Antreiben der Robotereinheit, um das Werkzeug in einer Weise zu bewegen, die direkt Steuereingaben von dem Bediener über die Steuerkomponente entspricht.

6. Einrichtung nach Anspruch 5, wobei der Computerprozessor dazu konfiguriert ist, zwischen dem realen Werkzeugmodus und einem virtuellen Werkzeugmodus zu schalten, wobei der Computerprozessor die Steuereingaben als indikativ für das Bewegen des virtuellen Werkzeugs interpretiert.

7. Einrichtung nach Anspruch 6, wobei der Computerprozessor dazu konfiguriert ist, beim Schalten in den virtuellen Werkzeugmodus einen Vorgang ausgewählt aus der Gruppe von Vorgängen bestehend aus Folgendem durchzuführen: Verwischen des Werkzeugs auf der Anzeige, teilweises Transparentmachen des Werkzeugs auf der Anzeige und digitales Entfernen des Werkzeugs von der Anzeige.

8. Einrichtung nach einem der Ansprüche 1 und 3-7, wobei der Computerprozessor dazu konfiguriert ist, eine entfernte Bewegungsmitte des Werkzeugs beizubehalten, wenn die entsprechende Spitze des Werkzeugs bewegt wird.

9. Einrichtung nach Anspruch 8, wobei der Computerprozessor dazu konfiguriert ist, die entfernte Bewegungsmitte durch Antreiben der Robotereinheit beizubehalten, um einen Teil des Werkzeugs zu steuern, der proximal zu der Spitze des Werkzeugs ist, um die entfernte Bewegungsmitte zu definieren, wenn die Spitze des Werkzeugs bewegt wird.

10. Einrichtung nach Anspruch 9, wobei der Teil des Körpers des Patienten ein Auge des Körpers des Patienten beinhaltet, und wobei der Computerprozessor dazu konfiguriert ist, die Robotereinheit anzutreiben, um den Teil des Werkzeugs innerhalb einer Einschnittsregion innerhalb einer Hornhaut des Patienten zu halten, wenn die Spitze des Werkzeugs bewegt wird.

11. Einrichtung nach einem der Ansprüche 1-9, wobei der Computerprozessor dazu konfiguriert ist, die Robotereinheit anzutreiben, um das Werkzeug zu bewegen, ohne eine Ausrichtung des Werkzeugs einzustellen, um der Ausrichtung des virtuellen Werkzeugs zu entsprechen.

12. Einrichtung nach einem der Ansprüche 1, 3-9 und 11, wobei:
das virtuelle Werkzeug im Wesentlichen senkrecht zu einer Oberfläche des Teils des Körpers des Patienten innerhalb des Bildes ausgerichtet ist und
das Werkzeug in einem Winkel in Bezug auf die Oberfläche des Körpers des Patienten ausgerichtet ist.

13. Einrichtung nach Anspruch 12, wobei:
der Teil des Körpers des Patienten ein Auge des Patienten beinhaltet,
das virtuelle Werkzeug im Wesentlichen senkrecht zu einer Mitte einer Hornhaut des Auges des Patienten innerhalb des Bildes ausgerichtet ist und
das Werkzeug in dem Winkel in Bezug auf die Hornhaut des Auges des Patienten ausgerichtet ist.

14. Computersoftwareprodukt, umfassend ein greifbares nichtflüchtiges computerlesbares Medium, auf dem Programmanweisungen gespeichert sind, wobei die Anweisungen, wenn durch einen Computerprozessor (28) gelesen, den Computerprozessor zu Folgendem veranlassen:
Antreiben einer Anzeige (24), um ein virtuelles Werkzeug (100) anzuzeigen, das eine virtuelle Darstellung eines Werkzeugs (21) ist, das durch eine Robotereinheit (20) in einer gegebenen Ausrichtung in Bezug auf einen Teil eines Körpers eines Patienten gehalten wird, überlagert auf einem Bild des Teils des Körpers des Patienten, wobei die virtuelle Darstellung zweckmäßigerweise derart geformt ist, dass sie auf Weisen bewegt werden kann, die bei Standardchirurgie möglicherweise nicht möglich sind, sodass zumindest eine Spitze des virtuellen Werkzeugs mit einer entsprechenden Spitze des Werkzeugs zusammenfällt, aber sich eine Ausrichtung des virtuellen Werkzeugs von der gegebenen Ausrichtung unterscheidet,
Empfangen einer Eingabe von einem Bediener (25), die angibt, dass die Spitze des virtuellen Werkzeugs auf eine gegebene Weise bewegt werden sollte, und
als Reaktion auf das Empfangen der Eingabe Antreiben der Robotereinheit, um das Werkzeug zu bewegen, sodass sich die entsprechende Spitze des Werkzeugs in Bezug auf den Teil des Körpers des Patienten entsprechend der gegebenen Weise bewegt.

## Revendications

1. Appareil (10) permettant d'effectuer une procédure sur une partie du corps d'un patient, l'appareil comprenant :
un dispositif d'imagerie (22) configuré pour imager la partie du corps du patient ;
un dispositif d'affichage (24) configuré pour afficher l'image de la partie du corps du patient à un opérateur (25) ;
une unité robotique (20) configurée pour maintenir un outil (21) suivant une orientation donnée par rapport à la partie du corps du patient ; et
au moins un processeur informatique (28) configuré pour :
commander au dispositif d'affichage d'afficher un outil virtuel (100), qui est une représentation virtuelle de l'outil qui est façonnée de manière pratique de sorte qu'elle puisse être déplacée d'une manière qui peut ne pas être possible en chirurgie standard, superposée sur l'image de la partie du corps du patient, de sorte qu'au moins une pointe de l'outil virtuel coïncide avec une pointe correspondante de l'outil mais qu'une orientation de l'outil virtuel diffère de l'orientation donnée,
recevoir une entrée de l'opérateur indiquant que la pointe de l'outil virtuel doit être déplacée d'une manière donnée, et
en réponse à cela, commander à l'unité robotique de déplacer l'outil de sorte que la pointe correspondante de l'outil se déplace, par rapport à la partie du corps du patient, de façon correspondante à la manière donnée.

2. Appareil selon la revendication 1, ladite entrée indiquant que la pointe de l'outil virtuel doit être déplacée selon un motif circulaire de déplacement par rapport à une surface de la partie du corps du patient dans l'image, et ledit processeur informatique étant configuré pour commander à l'unité robotique de déplacer la pointe de l'outil selon le motif circulaire de déplacement.

3. Appareil selon la revendication 1, comprenant en outre un composant de contrôle, ledit composant de contrôle étant configuré pour être déplacé par l'opérateur, et ledit processeur informatique étant configuré pour recevoir le déplacement du composant de contrôle en tant qu'entrée indiquant que la pointe de l'outil virtuel doit être déplacée de la manière donnée.

4. Appareil selon la revendication 3, ledit composant de contrôle comprenant un joystick comprenant un outil de composant de contrôle orienté de manière à correspondre à l'outil virtuel.

5. Appareil selon l'une quelconque des revendications 3 et 4, ledit processeur informatique étant configuré pour :
recevoir une autre entrée indiquant que le processeur informatique doit contrôler le déplacement de l'outil en mode outil réel, et
en réponse à cela, commander à l'unité robotique de déplacer l'outil d'une manière qui corresponde directement aux entrées de contrôle de l'opérateur par l'intermédiaire du composant de contrôle.

6. Appareil selon la revendication 5, ledit processeur informatique étant configuré pour commuter entre le mode outil réel et un mode outil virtuel, dans lequel le processeur informatique interprète les entrées de contrôle comme indiquant le déplacement de l'outil virtuel.

7. Appareil selon la revendication 6, ledit processeur informatique étant configuré pour, lors du passage au mode outil virtuel, effectuer une opération sélectionnée dans le groupe d'opérations consistant à : flouter l'outil sur le dispositif d'affichage, rendre l'outil partiellement transparent sur le dispositif d'affichage, et retirer numériquement l'outil du dispositif d'affichage.

8. Appareil selon l'une quelconque des revendications 1 et 3 à 7, ledit processeur informatique étant configuré pour maintenir un centre de déplacement distant de l'outil lorsque la pointe correspondante de l'outil est déplacée.

9. Appareil selon la revendication 8, ledit processeur informatique étant configuré pour maintenir le centre de déplacement distant en commandant à l'unité robotique de contrôler une partie de l'outil, qui est proximale à la pointe de l'outil, de manière à définir le centre de déplacement distant lorsque la pointe de l'outil est déplacée.

10. Appareil selon la revendication 9, ladite partie du corps du patient comprenant un œil du corps du patient, et ledit processeur informatique étant configuré pour commander à l'unité robotique de maintenir la partie de l'outil à l'intérieur d'une zone d'incision à l'intérieur d'une cornée du patient lorsque la pointe de l'outil est déplacée.

11. Appareil selon l'une quelconque des revendications 1 à 9, ledit processeur informatique étant configuré pour commander à l'unité robotique de déplacer l'outil sans ajuster une orientation de l'outil de manière à se conformer à l'orientation de l'outil virtuel.

12. Appareil selon l'une quelconque des revendications 1, 3 à 9 et 11 :
ledit outil virtuel étant orienté sensiblement perpendiculairement à une surface de la partie du corps du patient dans l'image, et
ledit outil étant orienté suivant un angle par rapport à la surface du corps du patient.

13. Appareil selon la revendication 12 :
ladite partie du corps du patient comprenant un œil du patient,
ledit outil virtuel étant orienté sensiblement perpendiculairement à un centre d'une cornée de l'œil du patient dans l'image, et
ledit outil étant orienté suivant un angle par rapport à la cornée de l'œil du patient.

14. Produit logiciel informatique comprenant un support tangible non transitoire lisible par ordinateur dans lequel des instructions de programme sont stockées, lesquelles instructions, lorsqu'elles sont lues par un processeur informatique (28), amènent le processeur informatique à :
commander à un dispositif d'affichage (24) d'afficher un outil virtuel (100), qui est une représentation virtuelle d'un outil (21) qui est tenu par une unité robotique (20) suivant une orientation donnée par rapport à une partie du corps d'un patient, superposée sur une image de la partie du corps du patient, la représentation virtuelle étant façonnée de manière pratique de sorte qu'elle puisse être déplacée d'une manière qui puisse ne pas être possible en chirurgie standard, de sorte qu'au moins une pointe de l'outil virtuel coïncide avec une pointe correspondante de l'outil mais qu'une orientation de l'outil virtuel diffère de l'orientation donnée,
recevoir une entrée d'un opérateur (25) indiquant que la pointe de l'outil virtuel doit être déplacée d'une manière donnée, et
en réponse à la réception de l'entrée, commander à l'unité robotique de déplacer l'outil de sorte que la pointe correspondante de l'outil se déplace, par rapport à la partie du corps du patient, de façon correspondante à la manière donnée.
